(19) Europäisches Patentamt
European Patent Office
Office européen des brevets

(11) EP 1 980 240 A1

(12) EUROPEAN PATENT APPLICATION

(43) Date of publication:
**15.10.2008 Bulletin 2008/42**

(21) Application number: **07290443.6**

(22) Date of filing: **11.04.2007**

(51) Int Cl.:
***A61K 9/00*** (2006.01)
***A61K 9/19*** (2006.01)
***A61K 9/14*** (2006.01)
***A61K 31/4178*** (2006.01)
***A61K 31/135*** (2006.01)
***A61K 31/4045*** (2006.01)
***A61K 38/11*** (2006.01)

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicants:
- **CEPHALON FRANCE**
  **94700 Maisons-Alfort (FR)**
- **CEPHALON, INC.**
  **Frazer, PA 19355 (US)**

(72) Inventors:
- **Dulieu, Claire**
  **94370 Sucy en Brie (FR)**
- **Durfee, Steve**
  **Sandy, UT 84092 (US)**
- **Holl, Richard**
  **Park City, UT 84098 (US)**
- **Nguyên, Thanh-Tâm**
  **94450 Limeil-Brevannes (FR)**

(74) Representative: **Colombet, Alain André et al**
**Cabinet Lavoix**
**2, place d'Estienne d'Orves**
**75441 Paris Cedex 03 (FR)**

# (54) Lyophilized pharmaceutical compositions and methods of making and using same

(57) The present invention relates to a new lyophilized pharmaceutical composition capable of adhering to oral mucosal tissue for an extended period of time for delivering active pharmaceutical ingredient through the oral mucosal tissue using transmucosal absorption.

EP 1 980 240 A1

## Description

BACKGROUND OF THE INVENTION

Oral administration is one of the most common methods of administering pharmaceutical compositions. The pharmaceutical compositions are generally formulated as a solid dosage form (such as a tablet or capsule) or a liquid dosage form (such as a solution or syrup), and after being placed in the mouth, they are often swallowed.

One possible disadvantage of oral administration is that pediatric and geriatric patients may have difficulty swallowing solid dosage forms. The act of swallowing the medicine often requires fluids which may not be available. Liquid dosage forms are often bad tasting and inconvenient and may increase gastric volume and the possibility of nausea and vomiting.

Moreover, there may be a substantial delay between oral administration and the therapeutic effect because the active pharmaceutical ingredient ("API") must pass through the gastrointestinal system in order to enter the blood stream; this typically takes 45 minutes or longer. In addition, API absorption through the digestive tract can be inefficient and can result in high loss due to, for example, food effects and first pass metabolism. The result is that some APIs are impracticable for oral administration, particularly many central nervous system and many cardiovascular-acting APIs that are intended for rapid onset in critical care situations.

In order to avoid some of the disadvantages of oral administration, injection or IV administration may be used. Injecting an API (generally intravenously), results in rapid entry of the API into the patient's blood stream. In addition, this type of delivery avoids first pass metabolism. As a result, when an API is administered parenterally, less API may be needed compared to oral administration.

However, most patients have an aversion to injections. In some patients, this aversion may be so pronounced as to make the use of injections a serious concern. Moreover, in all but unusual situations, a patient cannot self-medicate using injection or IV.

Sublingual, gingival and buccal administration of a drug is another oral method of administering pharmaceutical compositions. Sublingual, gingival and buccal medications are administered by placing them in the mouth, either under the tongue (sublingual), on gum tissue (gingival) or between the gum and the cheek (buccal). The medications dissolve and are absorbed through the mucous membranes of the mouth, where they enter into the blood stream. The sublingual, gingival and buccal medications are often compounded in the form of small, quick-dissolving tablets, gels, ointments, creams, sprays, lozenges, or liquid suspensions. See U.S. Patent Nos. 6,200,604, 6,974,590 and Shin et al., Mucoadhesive and Physico-chemical Characterization of Carbopol-Poloxamer Gels Containing Triamcinolone Acetonide, 26(3) Drug Development and Industrial Pharmacy 307-312 (2000).

Lyophilized dosage forms also allow the administration of the medication through the oral mucosal tissues. See, *e.g.,* Lafon, U.S. Patent No. 4,616,047 (the '047 Patent), Nguyen et al., U.S. Patent No. 5,843,347 (the '347 Patent) and Blonde et al., U.S. Patent No. 3,855,712. These lyophilized dosage forms can be based on lyophilized emulsions such as that described in the '047 Patent or lyophilized microbeads which may then be coated with a host of compounds including polyacrylics and polymethacrylic acid esters ethyl/vinyl acetate copolymers, polymethylcyloxanes, polyacrylamides, PVP and polyvinyl acetate, polyactic and polyglycolic acids and copolymers thereof, polyurethanes, polypeptides and others as discussed in the '347 patent. In one embodiment, the coating produces a microporous semipermeable membrane allowing the contained material to dissolve wherein an osmotic pressure is created which expels the aqueous solution containing the API. These formulations can include various diluents, sweeteners and artificial sweeteners, as well as natural or synthetic flavorings and mixtures thereof. *See also* U.S. Patent Nos. 4,490,407 and 3,939,260.

One form of lyophilized dosage form for buccal administration has been described in Caffaggi et al., Preparation and Evaluation of a Chitosan Salt-Poloxamer 407 Based Matrix for Buccal Drug Delivery, 102 Journal of Controlled Release 159-169 (2005). This article describes a compressed lyophilized oral dosage form prepared by first lyophilizing a solution containing chitosan and Poloxamer 407, deconstructing the lyophilized product into small pieces, and then using compressive force of 30kN for 1 minute in the pieces to form a compressed dosage form.

However, some dosage forms, including the lyophilized dosage forms for sublingual, gingival and buccal administration, can have drawbacks. They may not maintain the correct position in the mouth with the result that some of the API is swallowed, thereby reducing the oral mucosal absorption. Moreover, using compressive force to produce a dosage form adds another set of processing steps and fundamentally changes the attributes of the lyophilized product. The present invention helps address these disadvantages.

SUMMARY OF THE INVENTION

Generally, the present invention relates to a lyophilized pharmaceutical composition capable of adhering to an oral mucosal tissue, delivering at least one API through transmucosal absorption, and methods of making and using same.

In accordance with one aspect of the invention, a lyophilized solid dosage form of the present invention comprises at least one API, a gelling agent, and a bioadhesive polymer. The dosage form preferably can adhere to an oral mucosal tissue for at least about 10 minutes up to about 180 minutes, more preferably about 15 minutes up to about 180 minutes, although it need not do so, and releases the API through the oral mucosal tissue for transmucosal absorption.

In accordance with another aspect of the invention, the lyophilized solid dosage form of the present invention comprises at least one API, a gelling agent, and a bioadhesive polymer, wherein the lyophilized solid dosage form adheres to an oral mucosal tissue to release the API through the oral mucosal tissue wherein the dosage form exhibits at least one of the following characteristics: a density is from about 100 mg/ml to about 900 mg/ml, a porosity is from about 10% to about 90%, and a hardness is from about 4,5 N (Newton) to about 100 N. The lyophilized solid dosage form of the present invention may also exhibit two or more of the above-mentioned characteristics.

In accordance with yet another aspect, a lyophilized solid dosage form of the present invention results from an emulsion comprising an oil and an emulsifying agent in addition to the bioadhesive and gelling agent when a hydrophobic and/or lipophilic API is used. Alternatively, when a hydrophilic API is used, a lyophilized solid dosage form of the present invention results from a suspension comprising water, instead of an oil and an emulsifying agent, mixed with all other ingredients, including a gelling agent and a bioadhesive polymer.

In one preferred aspect of the invention, there is provided a method for preparing a lyophilized solid dosage form for transmucosal delivery of at least one hydrophilic API comprising the steps of: (a) preparing a suspension of a gelling agent, a bioadhesive polymer, and an API, (b) depositing the suspension into a mould, such as preformed blisters, (c) freezing and then lyophilizing the suspension in the mould to form a lyophilized solid dosage form, and (d) sealing the solid dosage form in the mould. As used herein, the term "suspension" also encompasses a solution, dispersion, emulsion, gel and the like. In accordance with the present invention, as long as the ingredients, such as a gelling agent, a bioadhesive polymer and an API, are mixed in a solvent in a substantially homogeneous manner, it is not critical that these ingredients are fully or partly suspended, dissolved, dispersed, emulsified, gelled and the like in the solvent before the lyophilization step.

In yet another preferred aspect of the invention, there is provided a method of preparing a lyophilized solid dosage form for transmucosal delivery of at least one lipophilic API comprising the steps of: (a) preparing an aqueous suspension containing at least one hydrophilic compound, such as a gelling agent, and a bioadhesive polymer, (b) preparing an oil phase suspension containing at least one lipophilic API and at least one other lipophilic compound, such as an emulsifying agent, (c) mixing the suspensions from steps (a) and (b) to form an emulsion, (d) depositing the emulsion from step (c) into a mould, such as preformed blisters, (e) freezing and then lyophilizing the emulsion of step (d) in the mould to form a lyophilized solid dosage form, and (f) sealing the solid dosage form in the mould.

In accordance with yet another aspect of the invention, there is also provided a method of treating a patient in need thereof comprising the steps of: (a) placing any of the lyophilized solid dosage forms described above as part of the invention, containing a therapeutically effective amount of at least one API, into intimate contact with an oral mucosal tissue of the patient; and (b) allowing the lyophilized solid dosage form to adhere to the oral mucosa and release the therapeutically effective amount of the at least one API through the oral mucosal tissue.

By employing the present invention which allows for the transmucosal absorption of the API, the API may be introduced into the patient's blood stream much faster than using the oral administration route when the dosage form is swallowed, while avoiding the negative aspects of such methods.

Furthermore, the present invention may also allow for sustained release and optimized bioavailability. The present invention is capable of adhering to the oral mucosal tissue for about 10 minutes up to about 180 minutes, more preferably about 15 minutes to about 180 minutes allowing a sustained release of API and avoidance of first pass metabolism. Alternatively, the present invention can also be used to deliver the API locally to treat conditions of the oral mucosal tissue.

In addition, by not using compressive forces in preparation of the lyophilized dosage forms of the present invention as performed by certain prior methods, the dosage forms of the present invention allow for faster dissolution, optimized bioavailability, rapid onset of action, uniform distribution of the API and improvement in stability.

In accordance with yet another aspect of the invention, a lyophilized solid dosage form of the present invention comprises a mixture of non-coated and coated API. This results in a controlled and/or extended release of API since the non-coated API will quickly enter the blood stream via transmucosal absorption, whereas the coated API will enter the blood stream at a much later time via systemic absorption.

In sum, the unique dosage form of the present invention is convenient and easy to use without water. It offers safe administration combined with rapid onset of action, sustained, controlled and/or extended release, which will result in better compliance and improvement of therapeutic efficacy.

## DETAILED DESCRIPTION

While the specification concludes with the claims particularly pointing and distinctly claiming the invention, it is believed that the present invention will be better understood from the following description. All percentages and ratios used herein are by weight of the total composition and all measurements made are at 25°C and normal pressure unless otherwise designated. All temperatures are in degrees Celsius unless specified otherwise. The present invention can comprise (open ended) or consist essentially of the components of the present invention as well as other ingredients or elements described herein. As used herein, "comprising" means the elements recited, or their equivalent in structure or function, plus any other element or elements which are not recited. The terms "having" and "including" are also to be construed as open ended unless the context suggests otherwise. As used herein, "consisting essentially of" means that the invention may include ingredients in addition to those recited in the claim, but only if the additional ingredients do not materially alter the basic and novel characteristics of the claimed invention. Preferably, such additives will not be present at all or only in trace amounts. However, it may be possible to include up to about 10% by weight of materials that could materially alter the basic and novel characteristics of the invention as long as the utility of the compounds (as opposed to the degree of utility) is maintained. All ranges recited herein include the endpoints, including those that recite a range "between" two values. Terms such as "about," "generally," "substantially," and the like are to be construed as modifying a term or value such that it is not an absolute, but does not read on the prior art. Such terms will be defined by the circumstances and the terms that they modify as those terms are understood by those of skill in the art. This includes, at very least, the degree of expected experimental error, technique error and instrument error for a given technique used to measure a value.

Note that while the specification and claims may refer to a tablet or other dosage form of the invention as, for example, containing particles having a certain particle size or distribution, it may be difficult to tell from the final dosage form that the recitation is satisfied. However, such a recitation may be satisfied if the materials used prior to final blending and tablet formulation, for example, meet that recitation. Indeed, as to any property of a dosage form which cannot be ascertained from the dosage form directly, it is sufficient if that property resides in the formulation just prior to producing a dosage form therefrom.

In describing the preferred embodiments of the present invention, specific terminology will be resorted to for the sake of clarity. However, the invention is not intended to be limited to the specific terms so selected, and is understood that each specific term includes all technical equivalence which operate in a similar manner to accomplish the same purpose.

The term "dosage form(s)" is defined as solids such as tablets, caplets, wafers, films and the like which result from lyophilization and are adapted to adhere to the oral mucosal tissue.

The term "oral mucosa" and "oral mucosal tissue" in accordance with the present invention includes the mucous membrane that covers all structures inside the oral cavity except the teeth, including the tissue under the tongue, gum tissue, between the gum and the cheek, and cheek pouch.

The term "sustained release" in this disclosure means that the dosage form begins its release and continues that release over an extended period of time, that is, from about 10 minutes up to about 180 minutes, more preferably about 15 minutes to about 180 minutes. Release can occur beginning almost immediately or can be delayed. Release can be constant, can increase or decrease over time, can be pulsed, can be continuous or intermittent, and the like.

"Controlled release" in accordance with the present invention means a release of the active pharmaceutical ingredient ("API") at a particular desired point in time once the dosage form has been placed into the mouth of a patient. Generally, it involves a delayed but otherwise complete release. A sudden and total release in the stomach at a desired and appointed time or a release in the intestines such as through the use of an enteric coating, are both considered controlled release, as opposed to a passive release dosage form wherein within moments of reaching the stomach, the API begins to be exposed to digestion and possibly the absorption process (such as occurs when swallowing a raw powdered API). Controlled release requires that release occur at a predetermined time or in a predetermined place within the digestive tract. It is not meant to be a passive, uncontrolled process as in swallowing a normal tablet.

An "extended release" dosage form is a dosage form which generally begins its release and continues that release over an extended period of time. Release can occur beginning almost immediately or can be delayed, in which case the extended release dosage form is also a controlled release dosage form as described above. Release can be constant, can increase or decrease over time, can be pulsed, can be continuous or intermittent, and the like. Generally, however, the release of the API from an extended release dosage form will exceed the amount of time of release of the drug taken as a normal, passive release tablet. Thus, for example, while all of the API of an uncoated aspirin tablet should be released within, for example, four hours, an extended release dosage form could release a smaller amount of aspirin over a period of six hours, 12 hours, or even longer. Extended release in accordance with the present invention generally means that the release occurs for a period of six hours or more, and more preferably 12 hours or more.

The term "oil" in this disclosure includes anything that can form an oil phase in an emulsion.

As used herein, the term "mould" refers to any containment of any shape and size suitable for the dosage form prepared according to the invention and capable of accommodating said dosage form during its preparation.

Active pharmaceutical ingredient(s) ("API(s)") which may be used in accordance with the present invention include materials that are biologically or therapeutically active, capable of being lyophilized and are desirable for transmucosal administration. The APIs in accordance with the present invention may include systematically distributable APIs, vitamins, minerals, dietary supplements, or mixtures thereof, as well as nonsystematically distributable APIs. In this disclosure, API may be comprised of either a mixture of non-coated and coated API, or non-coated API alone.

Generally, coating of an API can be accomplished using, for example, a Wurster fluidized bed where the coating material enters from the bottom of the reactor. See also the techniques described in U.S. Patent Nos. 6,024,981, 4,949,588, 5,178,878, and 5,055,306. See also Lieberman, Pharmaceutical Dosage Form: Tablets Vol. 1 (2d ed. 1989), 732-36. Other coating techniques contemplated by the present invention include spray drying, spraying chilling, spray congealing, hot melt coating in fluid beds or through extrusion, fluid bed top spray mode, fluid bed tangential spray mode, conventional pan coating, perforated pan coating, microencapsulation through coacervation or other forms of phase separations, interfacial polymerization, super critical fluid coating, spinning disc coating and other centrifugation coating techniques.

In this disclosure, the term "coating" is used broadly to include any material mixed, granulated, agglomerated, deposited on or coated with the API.

Generally, the coating is made from any natural or synthetic materials including: acrylic polymers, modified celluloses, cellulosic polymers, lactic acid polymers, glycolic acid polymers and copolymer thereof, and the like, fatty acids, fatty acid esters, fatty alcohols, phospholipids, hard fat, waxes, hydrogenated vegetable oils and the like.

These natural or synthetic materials can be pH dependant materials. In one embodiment, the coating materials will become soluble at a pH which is generally acidic (pH 7 or below) and in another embodiment, the coating materials will become soluble at a pH which is generally basic (pH 7 or above). In a third embodiment, the materials become soluble at a generally neutral pH (pH 6-8). There are a number of factors which determine which material is used as a coating in a given situation including, without limitation, ease of handling and processing, cost, thickness, site of intended dissolution of the coating and/or the API, and the type and pH of the lyophilization solvent to be used.

In one preferred embodiment, the coating is made from a material that becomes soluble at a pH of about 6.5 or below. In another embodiment, the coating becomes soluble at a pH of between about 6.0 and about 6.5. These polymers and copolymers should preferably be pharmacologically acceptable, capable of providing appropriate release and while still being convenient to process. These include, for example, acrylic polymers, modified cellulose amino alkyl acrylate copolymers such as, for example, copolymers of methylmethacrylate, butylmethacrylate and dimethyl-aminoethyl methacrylate.

A particularly preferred material can be obtained under the mark Eudragit® E-100, which can be used in normal form or in micronized Eudragit® E-100 and mixtures thereof. Eudragit® is a trademark of Rohm GmbH, Chemische Fabrik, Kirschenallee, D-64293, Darmstadt, Germany for a group of acrylic polymers.

These materials are generally solid at room temperature. However, they may be applied by being dissolved, suspended, emulsified, dispersed or the like in a solvent or solvent system, such as water or a supercritical fluid.

The coated APIs may be in the form of, without limitation, grains, microparticles, crystals, granules (wet or dry granulation), microgranules, agglomerates, pellets, mini-tablets, beads, solid supports, microcrystals and powders.

APIs include, without limitation, antalgic, anesthetic, antacids, analgesics, antiinflammatories, antibiotics, antidiabetes, diuretics, anorexics, antihistamines, antiasthmatics, antidiuretics, antiflatuents, antimigraine agents, antispaspodics, sedatives, antihyperactives, antihypertensives, tranquilizers, decongestants, immunosuppressants, anticancers, antivirals, antiparasitics, antifungals, antiemetics, antidepressants, antiepileptics, local anesthetics, vasoactive agents, antiasthmatics, skeletal muscle relaxants, drugs for parkinsonism, antipsychotics, hematopoietic growth factors, antihyperlipidemics, anticoagulants, fibrinolytics, chemotherapeutic agents, antithrombotics, hormones, therapeutic proteins and peptides, antiarrhythmia, antiangina, beta blockers, calcium inhibitors, drugs intended for management of moderate to sever pain when a continuous analgesic is needed for an extended period of time, drugs for treatment of anxiety, depression, emesis, migraine, panic attack, attention deficit hyperactivity disorder (ADHD), seizure, and cardiovascular disorders and mixtures thereof. Of these, the preferred APIs are antalgics, anaesthetics, antiinflammatories, chemotherapeutic agents, proteins and peptides, drugs intended for management of moderate to severe pain when a continuous analgesic is needed for an extended period of time, drugs for treatment of anxiety, depression, emesis, migraine, panic attack, attention deficit hyperactivity disorder (ADHD), narcolepsy, fatigue, apnea, seizure, cardiovascular disorders, betablocker, and calcium inhibitors. Of these, the most preferred APIs are fentanyl, modafinil, ondansetron, granisetron, sumatriptan, tramadol, desmopressin, calcitonin, insulin, GLP-1, PPY, oxycodone, taxol, clarithromycin, erythromycin, azithromycin, amoxicillin, fexofenadine, ibuprofen, phenitol, acyclovir, famciclovir, DOPA, and L-DOPA and as well as their salts.

As used in this disclosure, the term "vitamin" refers to organic substances that are required in the diet. For the purposes of the present invention, vitamin(s) include, without limitation, thiamin, riboflavin, nicotinic acid, pantothenic acid, pyridoxine, biotin, folic acid, vitamin B12, lipoic acid, ascorbic acid, vitamin A, vitamin D, vitamin E and vitamin K. Also included within the term vitamin are the coenzymes thereof. Coenzymes are specific chemical forms of vitamins. Coenzymes that may be useful in the present invention include thiamine pyrophosphates (TPP), flavin mononucleotide (FMM), flavin adenine dinucleotide (FAD), nicotinamide adenine dinucleotide (AND), nicotinamide adenine dinucleotide phosphate (NADP) coenzyme A (CoA) pyridoxal phosphate, biocytin, tetrahydrofolic acid, coenzyme $B_{12}$, lipoyllysine,

11-cis-retinal, and 1,25-dihydroxycholecalciferol. The term vitamin(s) also includes choline, carnitine, and alpha, beta, and gamma carotenes.

As used in this disclosure, the term "mineral" refers to inorganic substances, metals, and the like required in the human diet. Thus, the term "mineral" as used herein includes, without limitation, calcium, iron, zinc, selenium, copper, iodine, magnesium, phosphorus, chromium and the like and mixtures thereof.

While having one API is desired, multiple APIs may also be used. The amount of API used can vary greatly and can depend upon, among other things, the type and properties of the API, the condition it is intended to treat, the method of administration, the size and type of the patient, the size and nature of the dosage form, whether or not more than one API is to be delivered from the dosage form and how many dosage forms will be used to deliver each dose.

Generally, the total amount of API for any individual dosage form is a therapeutically effective amount. The term "therapeutically effective amount" is the amount or quantity of a drug or API which is sufficient to elicit the required or desired therapeutic response, or in other words, the amount which is sufficient to elicit an appreciable biological response when administered to a patient. This does not need to be optimal, nor even provide a cure or symptomatic relief. As used with reference to a vitamin or mineral, the term "effective amount" means an amount at least about 10% of the United States Recommended Daily Allowance ("RDA") of that particular ingredient for a patient. For example, if an intended ingredient is vitamin C, then an effective amount of vitamin C would include an amount of vitamin C sufficient to provide 10% or more of the RDA.

In general, the total amount of API in a single dosage form is in an amount of about 0.001 mg to about 1.5 g by weight, more preferably about 0.01 mg to about 0.5 g, even more preferably about 0.02 mg to about 200 mg, and most preferably about 0.05 mg to about 100 mg. This is based on the amount of API only - not counting, for example, the amount of other excipients, coatings and the like in the final dosage form.

In terms of the proportion of the API with respect to the total weight of the dosage form, the amount of API can range from about 0.001% to about 70% by weight, and more preferably from about 0.1% to about 30% by weight relative to the total weight of the dosage form.

The APIs may be hydrophilic, hydrosoluble, lipophilic or liposoluble. In this disclosure, the term "hydrosoluble" is used interchangeably with the term "hydrophilic." Similarly, the term "liposoluble" is used interchangeably with the term "lipophilic." Moreover, the term "hydrophobic" is used interchangeably with the terms "liposoluble," lipohilic," and "water insoluble" in this disclosure.

The average particle size of hydrophilic APIs ranges from 10 μm to 800 μm, more preferably from 100 μm to 500 μm. The particle size is measured by sieving based on a determination by weight using the following screens: 1000, 710, 500, 355, 250, 180, 125, 90 μm and the pan. In these instances, no more than about 35% by weight would go through a 90 μm screen. The particle size of the APIs is measured before being exposed to a liquid used in the manufacturing process. The particle size of less than 20 μm is measured by laser light scattering using for example a Coulter LS230.

The average particle size of lipophilic APIs ranges from 1 μm to 100 μm, more preferably from 2 μm to 20 μm. The particle size is measured by laser light scattering, using for example a Coulter LS230.

Gelling agents in accordance with the present invention include materials capable of forming a gel and make the solution, suspension or emulsion viscous before the lyophilization step. Such gelling agents are used to avoid aggregation, agglomeration or cohesion of small insoluble API particles before lyophilization. Gelling agents are also often solvent swellable when in contact with water or saliva in the mouth. They provide a large adhesive surface for maximum contact with the oral mucosal tissue. Also, presence of these gelling agents confers a gel-like structure upon hydration of the dosage form in the mouth. This gel-like structure is useful for maintaining the dosage form during the time needed for permeation of drugs through the mucosa. Moreover, this gel-like structure gets the dosage form softer when in place on the oral mucosa, what is comfortable for the patient.

Such gelling agents may include, without limitation, acacia, alginic acid, bentonite, carbomer, carboxymethylcellulose sodium, cetostearyl alcohol, colloidal silicon dioxide, ethylcellulose, gelatin, hydroxyethylcellulose, hydroxypropyl cellulose, hydroxypropyl methylcellulose, magnesium aluminum silicate, maltodextrin, methylcellulose, polyvinyl alcohol, povidone, propylene carbonate, propylene glycol alginate, sodium alginate, sodium starch glycolate, starch tragacanth, polysaccharides, such as colloid, hydrocolloid, guar gum, arabic gum, tragacanth gum, xanthan gum, carraghenans, pectins, starch, and specialized polymers such as poloxamer 188, poloxamer 407, poloxamine, acrylic polymers, carbopol and mixtures thereof.

Of these, the preferred gelling agents are colloids or hydrocolloids of polysaccharides, such as, guar gum, arabic gum, tragacanth gum, xantham gum, carraghenans, pectins, starch, and specialized polymers such as poloxamer 188, poloxamer 407, poloxamine, acrylic polymers, and carbopol.

The gelling agents can be used in conventional amounts and preferably in an amount of from about 0.5% to about 50% by weight, and more preferably from about 3% to about 30% by weight relative to the total weight of the dosage form.

A bioadhesive polymer in accordance with the present invention includes materials capable of adhering to a biological substrate and remaining there for an extended period of time to aid in sustained release of an API through oral mucosa membrane. The term, bioadhesive, in accordance with the present invention, includes compounds conventionally referred to as muco-adhesive, and these terms may be used interchangeably herein.

Such bioadhesive polymers may include, without limitation, polycarbophil, polyacrylates, cellulose, cellulose derivatives, carboxymethyl cellulose, sodium carboxymethyl cellulose, methyl cellulose, hydroxypropyl cellulose, hydroxy propyl methyl cellulose, hydroxy ethyl cellulose, hyaluronic acid, sodium alginate, povidone, chitin, glycerol ester, pectin, gelatin, PEG and mixtures thereof.

Of these, the preferred bioadhesive polymer is cellulose, cellulose derivatives or mixtures thereof.

The bioadhesive polymer can be used in conventional amounts and preferably in an amount of from about 0.5% to about 30% by weight, and more preferably from about 1.0% to about 15% by weight relative to the total weight of the dosage form.

In some preferred embodiments, when the gelling agent used is poloxamer 407, the bioadhesive polymer is not chitosan and vice versa.

A solvent in accordance with the present invention includes water such as filtered, purified, distilled water or a mixture thereof.

In another preferred embodiment, a lyophilized solid dosage form of the present invention may further comprise an oil and an emulsifying agent, when the API is a lipophilic API. The emulsion can be an oil-in-water or a water-in-oil-in-water emulsion (double emulsion).

Oils are used in accordance with the present invention as a carrier of a hydrophobic and/or lipophilic API. Some oils that may be useful in the present invention include, without limitation, Miglyol® 810-818, mono, di-triglycerides esters, fatty acid of glycerol, a mineral oil, vegetable oil and a reformed vegetable oil of a known composition. Of these, the preferred oils are Miglyol® 810-818, mono-di-triglycerides esters, fatty acid esters of glycerol. Oils can be used in conventional amounts and preferably in an amount of from about 0.5% to about 40% by weight, and more preferably from about 2% to about 20% by weight relative to the total weight of the dosage form.

Emulsifying agents are used to prepare an oil-in-water or water-in-oil-in-water emulsion for lyophilization. Emulsifying agents are used in accordance with the present invention as wetting agents that lower the interfacial tension between oil and water, allowing easier spreading. Non-limiting examples of emulsifying agents that are suitable for the present invention are anionic, cationic, amphoteric, nonionic surfactants, such as dioctyl sulfosuccinate, sucroester 7, sucroester 11, sucroester 15, polysorbate 20, polysorbate 60, polysorbate 80; poloxamers 188, 407, sorbitan stearate, polyethoxyethers of fatty glycerides, cetyl ethers of polyoxyethylenglycol, palmitostearate of polyoxyethyleneglycol, lecithins, and mixtures thereof. Some emulsifying agents that may be useful in the present invention include, without limitation, various grades of the following commercial products: Arlacel®, Tween®, Capmul®, Centrophase®, Cremophor®, Labrafac™, Labrafil®, Labrasol™, Myverol™, Tagat®, Simulsol®, Solutol®, Softigen® and any non-toxic short and medium chain alcohols. Of these, the preferred emulsifying agents are polysorbate 20, polysorbate 60, polysorbate 80, sucroester 7, sucroester 11, sucroester 15, sorbitan, poloxamer and dioctyl sulfosuccinate, laurylsulfate. Emulsifying agents can be used in conventional amounts and preferably in an amount of from about 0.01% to about 20% by weight, and more preferably from about 0.1 % to about 10% by weight relative to the total weight of the dosage form.

The lyophilized dosage form of the present invention may further comprise at least one excipient, which is generally a pharmaceutically inactive substances added to the formulation to facilitate tableting or lyophilization. Generally, lyophilized dosage forms of the present invention may contain excipients for various reasons, such as to bulk up the amount of solids, to buffer the product, to taste-mask the bad or bitter tasting APIs, to facilitate the process by avoiding foam forming, to increase the rate at which the APIs permeate through the oral mucosal tissue and enters the blood stream and/or to protect the API from the adverse effects of freezing and/or drying, etc. Examples of some lyophilization excipients used in accordance with the invention, without limitation, are binders, fillers, sweeteners, flavoring agents, permeation enhancers, buffers, coloring agents, defoaming agents, mixtures thereof, and the like. Of these, binders and fillers are hydrophilic excipients. Sweeteners, flavoring agents, coloring agents, defoaming agents and permeation enhancers may be either hydrophilic or lipophilic, depending on the specific compound.

Binders and fillers are used to contribute to the bulk and stability of the matrix and control the rate at which the matrix will dissolve. Binders can be anything known to be used as a binder. Binders that may be useful in the present invention include Dextran 70, povidone 12, povidone 17 or povidone 30, copovidone, gelatin, starch, pregelatinized starch, methyl cellulose, hydroxypropylmethyl cellulose and mixtures thereof. Binders can be used in conventional amounts and preferably in an amount of from about 0.2% to about 20% by weight, and more preferably from about 2% to about 10% by weight relative to the total weight of the dosage form.

Fillers can be anything known to be useful as fillers. Some fillers that may be useful in the present invention include mannitol, lactose, sorbitol, isomalt, glycine, cyclodextrins and derivatives, glucose, sucrose maltodextrine, trehalose. Fillers can be used in conventional amounts and preferably in an amount of from about 0.5% to about 99% by weight, and more preferably from about 5% to about 50% by weight relative to the total weight of the dosage form.

Permeation enhancers as used herein are materials that can increase the rate at which the API permeates through the oral mucosal tissue and enters the blood stream. Such permeation enhancers may include, without limitation, sodium salicylate, sodium dodecyl sulfate, citric acid, sodium bicarbonate, sodium carbonate, sodium glycolate, sodium deoxycholate, sodium taurocholate, fatty acids, Azone®, chitosan and its derivatives, terpenoids, protease inhibitors and a mixture thereof. Permeation enhancers can be used in conventional amounts and preferably in an amount of from about 0.001 % to about 10% by weight, and more preferably from about 0.01 % to about 5% by weight relative to the total weight of the dosage form.

Buffers as used herein are materials capable of adjusting pH in the preparation for the purposes of stability, permeation and/or taste. Some buffers that may be useful in the present invention include any weak acid or weak base or, preferably, any buffer system that is not harmful to the oral mucosa and gastrointestinal system. These include, but are not limited to, any of the acids or bases such as monobasic potassium phosphate, anhydrous dibasic sodium phosphate, sulfuric acid, sodium hydroxide, sodium carbonate, potassium carbonate, disodium hydrogen phosphate, sodium dihydrogen phosphate, and the equivalent potassium salts. Of these, the preferred buffers are monobasic potassium phosphate, anhydrous dibasic sodium phosphate, sulfuric acid, sodium hydroxide, citric acid, sodium citrate, acetic acid, sodium acetate, sodium carbonate, sodium bicarbonate or a mixture thereof. Buffers can be used in conventional amounts and preferably in an amount of from about 0.1 % to about 10% by weight, and more preferably from about 0.2% to about 5% by weight relative to the total weight of the dosage form.

Sweeteners include both natural and artificial sweeteners that are known to be and can be used as sweeteners. Some natural sweeteners that may be useful in the present invention, without limitation, include sucanat, (unpasteurized) honey, frozen juice concentrates, dates, raisin, fructose, and mixtures thereof. Some artificial sweeteners that may be useful in the present invention, without limitation, include saccharin such as sodium saccharin, cyclamate, aspartame, sucralose, neotame, and acesulfam potassium and polyols. Sweeteners may be used in conventional amounts, and preferably in an amount ranging from about 0.1% to about 20% by weight, and more preferably from about 0.5% to about 10% by weight relative to the total weight of the dosage form.

Flavoring agents can be anything known to be useful as flavoring agents. Flavoring agents that may be useful in the present invention may include synthetic flavor oils and flavoring aromatics and/or natural oils, extracts from plants, leaves, flowers, fruits and mixtures thereof. These may include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil and mixtures thereof. Also useful as flavoring agents are vanilla, citrus oil, including lemon, orange, banana, grape, lime and grapefruit, and fruit essences, including apple, pear, peach, strawberry, raspberry, cherry, plum, pineapple, apricot and mixtures thereof. Flavoring agents may be used in conventional amounts, and preferably in an amount ranging from about 0.1 % to about 20% by weight, and more preferably from about 1% to about 10% by weight relative to the total weight of the dosage form.

Coloring agents can be anything known to be used as a coloring agent. Coloring agents useful in the present invention may include titanium dioxide, and dyes suitable for food such as those known as F. D. & C. dyes and natural coloring agents such as grape skin extract, beet red powder, beta-carotene, annatto, carmine, turmeric, paprika, and mixtures thereof. Coloring agents may be used in conventional amounts, and preferably in an amount ranging from about 0.01% to about 5% by weight, more preferably from about 0.05% to about 2% by weight relative to the total weight of the dosage form.

Defoaming agents can be any substance used to reduce or inhibit foam formation caused by, for example, bioadhesive polymers, emulsifying agents and/or flavoring agents during the mixing process. Defoaming agents useful in the present invention may include silicone emulsions, simethicone, dimethicone copolyol, glyceryl oleate, propylene glycol, poloxamer (PO-EO block copolymers). Defoaming agents may be used in conventional amounts, and preferably in an amount ranging from about 0.001% to 5% by weight, more preferably from 0.005% to 3 % by weight relative to the total weight of the dosage form.

In some embodiments, the lyophilized solid dosage form of the present invention exhibits at least one of the following characteristics: the density is from about 100 mg/ml to about 900 mg/ml, the porosity is from about 10% to about 90%, and the hardness is from about 4.5 N(Newton) to about 100 N. In a more preferred embodiment, the lyophilized solid dosage form of the present invention exhibits at least two of the above-mentioned characteristics.

The lyophilized solid dosage form of the present invention is prepared without any step of breaking, cutting, milling or pulverizing the lyophilized product and/or compressing the broken, cut, milled or pulverized lyophilized materials into tablets by applying compressive force. This results in a lyophilized solid dosage form having, for example, a hardness of from about 4.5 N to about 100 N, allowing a much softer and flexible dosage form than compressed tablets of the prior art. The softness of the lyophilized solid dosage form of the present invention helps reduce the adverse feeling in the oral mucosa cavity on use and therefore results in better patient compliance.

The hardness of the lyophilized solid dosage form of the present invention is tested using a tablet hardness meter manufactured by Schleuniger Pharmatron. This is an electrically operated tablet tester that eliminates operator variability. The tests are performed with 10 tablets and the mean value is determined. Tablet hardness is represented by the force needed to crush the tablet (units kp or N). A larger number indicates a stronger tablet.

Moreover, the dosage forms of the present invention may have a porosity between about 10% to about 90%. This is also an advantage over some prior art dosage forms since it allows for fast dissolution, rapid gelling and adhesion, and rapid onset of action.

The porosity of the lyophilized solid dosage form of the present invention is calculated by the following equation:

$$\text{Porosity (\%)} = \frac{\text{Volume of water used prior to lyopilization}}{\text{Volume of the final dosage form after lyophilization}}$$

Even though the entire amount of water in the dosage form completely sublimes thereby leaving pores in the final dosage form, the volume of the dosage form after lyophilization does not change. Therefore, the porosity is determined by the percentage of water in the dosage form before lyophilization.

The lyophilized solid dosage form of the present invention may have a density ranging from about 100 mg/ml to about 900 mg/ml. During the lyophilization process, the volume of the dosage form remains unchanged. Therefore, the volume of the final dosage form is similar to the volume of the suspension before lyophilization.

Accordingly, the density of the final dosage form after lyophilization is calculated using the following equation:

$$\text{Density} = \frac{(\text{weight of suspension} - \text{weight of water})}{\text{Volume}}$$

In yet another preferred embodiment, the lyophilized solid dosage form of the present invention may be a controlled released and/or extended release formulation containing a mixture of coated and non-coated APIS. The non-coated APIs, which are explained in details above, will be absorbed through the mucous membranes of the mouth, where they enter into the blood stream, and therefore will be fast-acting. On the other hand, the coated APIs will be swallowed and passed through the gastrointestinal system in order to enter the blood stream, and therefore will take much longer to deliver therapeutic effect. Accordingly, a combination of coated and non-coated granules in the lyophilized solid dosage form of the present invention will result in an extended release of the API.

As long as a resultant dosage form meets the overall objectives of the present invention, and contains a therapeutically effective amount of at least one API, the shape or size of the dosage form is not critical. In fact, the dosage form can be of any shape, such as a circle, oval, rectangle, square, triangle, octagon and etc.

However, it is preferred that the lyophilized solid dosage form of the present invention has a maximum diameter of about 3 to about 40 mm and a thickness of about 1 to about 7 mm, preferably a maximum diameter of about 5 to about 20 mm and a thickness of about 1 to about 3 mm, as measured by a caliper rule.

In a preferred embodiment, a lyophilized solid dosage form of the present invention is prepared by first preparing a suspension containing a gelling agent and a bioadhesive polymer. This is preferably carried out by suspending, dissolving, or dispersing the bioadhesive polymer, preferably in water, at about 30°C-80°C, preferably 40°C-70°C, and then in a separate container, suspending, dissolving, or dispersing the gelling agent, preferably in water, at 10°C-30°C, preferably at 15°C-25°C, and mixing the two suspensions. The mixing can be performed at room temperature, preferably at 10°C-20°C since the solution is less viscous and allows easier mixing at a lower temperature. The remaining ingredients, such as an API, preferably hydrophilic API, filler, binder, sweetener, flavor, and/or permeation enhancer are added and mixed until the solution is substantially homogeneous. The aqueous solution, suspension, or dispersion is then poured into moulds shaped into a variety form, or preformed blisters (PVC or PVC/PVDC or Aclar and aluminum foil or AL/AL) and lyophilized. These are then loaded into a lyophilizer wherein the materials are first frozen and then lyophilized. Lyophilization is performed as described Lafon, U.S. Patent No. 4,616,047, Nguyen et al.*,* U.S. Patent No. 5,843,347, and Blonde et al., U.S. Patent No. 3,855,712, all of which are herein incorporated by reference. Typically, freezing of the preparation is performed at very low temperature, *i.e.,* -30°C to -45°C. The molecules of all the ingredients are immobilized and their properties remain unaltered as the rate of chemical reactions is nearly zero at this low temperature. Then the frozen preparations are lyophilized at low temperature and under low pressure where ice is directly converted into vapor phase. The lyophilization process is performed under a pressure within the range of about 5 to 500 μbar (4 X $10^{-2}$ mmHg to about 4 X $10^{-1}$ mmHg.) The secondary drying is operated at a temperature of about 20-60°C and a pressure within the range of about 20-50 μbar.

In another preferred embodiment, a lyophilized solid oral dosage form of the present invention containing a hydrophobic and/or lipophilic API is prepared by preparing an oil-in-water emulsion. This is carried out by first preparing an oil suspension or solution containing a lipophilic API and other lipophilic compounds, such as emulsifying agents, oils, flavoring agents, coloring agent and/or permeation enhancer. Then an oil-in-water emulsion is prepared by any process known to obtain an oil-in-water emulsion. The aqueous phase used to prepare the oil-in-water emulsion can contain all or part of the hydrophilic compounds necessary to obtain the dosage form, such as gelling agents, bioadhesive polymer, fillers, binders, sweeteners, coloring agents, buffers, and/or permeation enhancers. These two suspensions are then mixed until it is substantially homogeneous, poured into molds shaped into a variety form, or preformed blisters and lyophilized.

Unlike the prior art, the methods of making a lyophilized solid oral dosage form of the present invention preferably do not comprise any step of breaking, cutting, milling or pulverizing of the lyophilized oral solid dosage form and/or applying compressive force to form a compressed oral solid dosage form. Because the lyophilized solid oral dosage form of the present invention may be made without using any compressive force, it allows for faster dissolution, optimized bioavailability, rapid onset of action, uniform distribution of the API and improvement in stability. It also provides advantages in terms of the properties of the resulting dosage form including density, porosity and/or hardness. In addition, the method of the present invention does not include any step of granulation (*i.e.*, wet or dry granulation), simplifying the manufacturing process.

Once lyophilized, it is contemplated that the dosage forms may be stamped, painted, coated, printed, etc. These dosage forms may be stored in bulk, in blister packs, in conventional openable and reclosable multi-tablet bottles or other similar packaging. The preferred packaging is to store them in sealed blister packs.

In accordance with yet another aspect of the invention, there is also provided a method of treating a patient in need thereof comprising the steps of: (a) placing a lyophilized solid dosage form containing a therapeutically effective amount of at least one API into intimate contact with an oral mucosal tissue of the patient; and (b) allowing the lyophilized solid dosage form to dissolve and release the therapeutically effective amount of the at least one API through the oral mucosal tissue. Once the lyophilized dosage form of the present invention is placed on a specific location of the oral mucosal tissue, the gelling agent in the dosage form reacts with the saliva in the mouth and immediately swells by formation of an hydrated network. This swelling of the dosage form allows to provide a large adhesive surface for maximum contact with the oral mucosal tissue. Moreover, the network created by hydration of the dosage form in contact with the oral mucosa allows maintaining a physical structure compatible with a residence time of the dosage form in the mouth. The bioadhesive polymer, at the same time, helps the dosage form to remain at the correct position for an extended period of time to aid in the sustained release of an API through the oral mucosal tissue, thereby increasing the transmucosal absorption of the API, avoiding first pass metabolism.

The frequency of dosing depends on various factors including the amount of API present in the dosage form, the size of the dosage form, the weight of the patient, the condition of the patient, side effects of the API, etc. The administration of multiple dosage forms and multiple frequency of dosing is contemplated depending upon the above factors as well as duration of the patient's condition, how long the API stays in the patient's system, etc.

EXAMPLES 1-3

Examples 1 to 3 are matrix compositions (Placebo) without API aimed to incorporate different types of API depending upon their compatibility and characteristics. These placebos allowed the determination of bioadhesion of these compositions safely on human volunteers.

| Examples | | 1 | 2 | 3 |
|---|---|---|---|---|
| Components | | (mg) | (mg) | (mg) |
| API | | - | - | - |
| Gelling agent | Poloxamer 407 | 15 | 15 | 15 |
| Bioadhesive polymer | Methylcellulose (Methocel® A 15) | 5 | 10 | 15 |
| Binder | Dextran 70 | 10 | 10 | 5 |
| Filler | Isomaltidex | 10 | - | - |
| | Mannitol | 109 | 114 | 113 |
| Sweetener | Sucralose | - | - | 1 |
| API | Simethicone Q7 | 1 | 1 | 1 |
| Purified water | | 200 | 200 | 200 |

(continued)

| Examples | 1 | 2 | 3 |
|---|---|---|---|
| Components | (mg) | (mg) | (mg) |
| Total weight of the solid dosage form | 150 | 150 | 150 |
| Disintegration time of the solid dosage form (seconds) | 20 | 30-40 | 120 |
| Density of the solid dosage form (mg/ml) | 430 | 430 | 430 |
| Porosity of the solid dosage form (%) | 57 | 57 | 57 |
| Hardness of the solid dosage form (N) | 15-20 | 25-30 | 25-30 |
| Bioadhesion of the solid dosage form (min) | 15-30 | 60-120 | 90-180 |

The bioadhesive polymer, Methocel® A 15, was dissolved in water at 60-70°C, and the gelling agent, Poloxamer 407, was dissolved in water at 10-20°C in separate containers. Then a viscous water suspension containing the both the bioadhesive polymer and the gelling agent was prepared by mixing an aqueous solution containing Methocel® A 15 and an aqueous solution containing Poloxamer 407 at 15-25°C. Then, the remaining ingredients, which are antifoam agent (Simethicone Q7), binder (Dextran 70), fillers, (Isomaltidex and Mannitol) and sweetener (sucralose) were introduced to the viscous solution at 15-25°C. The suspension was mixed until it was substantially homogeneous and then deposited into preformed blisters.

The mixture was then frozen by means of a countercurrent of nitrogen and was cooled (in the freezing stage of a lyophilizer) to a temperature between -30°C to -35°C. The water is then removed from the frozen mixture by sublimation by lyophilizing at low temperature (-40°C to -45°C) and under low pressure (.300 mbar to 0.050 mbar) where ice was directly converted into a vapor phase.

The resulting lyophilized bioadhesive patch had an excellent mechanical strength, bioadhesive characteristics, and disintegration time.

Density, porosity and hardness were determined as explained above. Bioadhesiveness was measured by lab volunteers by applying the dosage form on the upper gum and measuring the wearing time. Disintegration time was determined with apparatus and method according to USP 701 in 6 individual unit doses.

EXAMPLES 4-6

Examples 4 to 6 are other matrix type compositions (*i.e.*, placebos without API).

| Examples | | 4 | 5 | 6 |
|---|---|---|---|---|
| Components | | (mg) | (mg) | (mg) |
| API | | - | - | - |
| Gelling agent | Poloxamer 407 | 10 | 15 | 18 |
| Bioadhesive polymer | Hypromellose (Methocel® E5) | 8 | 5 | 4 |
| Binder | Dextran 70 | 8 | 10 | 10 |
| Filler | Mannitol | 173 | 169 | 167 |
| Sweetener | Sucralose | 1 | 1 | 1 |
| Purified water | | 200 | 200 | 200 |
| Total weight of the solid dosage form | | 200 | 200 | 200 |
| Disintegration time of the solid dosage form (seconds) | | 20 | 20 | 30 |
| Density of the solid dosage form (mg/ml) | | 500 | 500 | 500 |
| Porosity of the solid dosage form (%) | | 50 | 50 | 50 |
| Hardness of the solid dosage form(N) | | 20-25 | 15 | 15 |
| Bioadhesion of the solid dosage form (min) | | 10-15 | 10-15 | 10-15 |

A mixture having the above formulations illustrated in Examples 4-6 was used to prepare a lyophilized bioadhesive dosage form and tested according to the process details described in connection with Examples 1-3 above.

The resulting lyophilized bioadhesive patch had an excellent mechanical strength, bioadhesive characteristics, and disintegration time.

EXAMPLES 7-8

Examples 7 and 8 are compositions intended for treating emesis before and after chemotherapy and radiotherapy.

| Examples | | 7 | 8 |
|---|---|---|---|
| Components | | (mg) | (mg) |
| API | Granisetron HCl | 1 | 1 |
| Gelling agent | Poloxamer 407 | 15 | 15 |
| Bioadhesive polymer | Methylcellulose (Methocel® A 15) | 10 | 8 |
| Binder | Dextran 70 | 8 | 10 |
| Filler | Mannitol | 115 | 115 |
| Sweetener | Sucralose | 1 | 1 |
| Purified water | | 200 | 200 |
| Total weight of the solid dosage form | | 150 | 150 |

The bioadhesive polymer, Methocel® A 15, is dissolved in water at 60-70°C, and the gelling agent, Poloxamer 407, is dissolved in water at 10-20°C in separate containers. Then, a viscous water suspension containing both the bioadhesive polymer and the gelling agent is prepared by mixing an aqueous solution containing Methocel® A 15 and an aqueous solution containing Poloxamer 407 at 15-25°C. Then, the remaining ingredients, which are API (Granisetron HCl), binder (Dextran 70), filler, (Mannitol) and sweetener (sucralose), are introduced to the viscous solution at 15-25°C. In this case, the API (Granisetron HCL) is fully dissolved in water. The solution is mixed until it is substantially homogeneous and then distributed into preformed blisters.

The mixture is then lyophilized according to the process details described in connection with Examples 1-3 above.

EXAMPLES 9-10

Examples 9 and 10 are compositions intended for treatment of emesis with rapid onset of action.

| Examples | | 9 | 10 |
|---|---|---|---|
| Components | | (mg) | (mg) |
| API | Ondansetron $HCl_2H_2O$ | 4 | 4 |
| Gelling agent | Poloxamer 407 | 15 | 15 |
| Bioadhesive | Hypromellose (Methocel® E5) | 8 | 8 |
| Binder | Dextran 70 | 10 | 10 |
| Filler | Mannitol | 113 | 53 |
| | β Cyclodextrin | - | 60 |
| Sweetener | Sucralose | 2 | 2 |
| Purified water | | 200 | 200 |
| Total weight of the solid dosage form | | 150 | 150 |
| Disintegration time of the solid dosage form (seconds) | | 20-30 | 20-30 |

(continued)

| Examples | 9 | 10 |
|---|---|---|
| Components | (mg) | (mg) |
| Density of the solid dosage form (mg/ml) | 420 | 420 |
| Porosity of the solid dosage form (%) | 58% | 58% |
| Hardness of the solid dosage form (N) | 15-25 | 10-20 |
| Bioadhesion of the solid dosage form (min) | Not Determined | Not Determined |

The bioadhesive polymer, Methocel® A 15, was dissolved in water at 60-70°C, and the gelling agent, Poloxamer 407, was dissolved in water at 10-20°C in separate containers. Then, a viscous water suspension containing both the bioadhesive polymer and the gelling agent was prepared by mixing an aqueous solution containing Methocel® A 15 and an aqueous solution containing Poloxamer 407 at 15-25°C. Then, the remaining ingredients, which are API (Ondansetron $HCl_2H_2O$), binder (Dextran 70), fillers, (Mannitol and β Cyclodextrin) and sweetener (sucralose), were introduced to the viscous solution at 15-25°C. In this case, the API (Ondansetron $HCl_2H_2O$) was dispersed in water. The solution was mixed until it was substantially homogeneous and then distributed into preformed blisters.

The mixture was then lyophilized and tested according to the process details described in connection with Examples 1-3 above.

The resulting lyophilized bioadhesive patch had an excellent mechanical strength, bioadhesive characteristics, and disintegration time.

Examples 11 and 12 are compositions intended for treating moderate to severe pain. A composition illustrated in Example 11 is a sustained release formulation.

| Examples | | 11 | 12 |
|---|---|---|---|
| Components | | (mg) | (mg) |
| API | Tramadol chlorhydrate | 50 | 50 |
| Gelling agent | Poloxamer 407 | 15 | 15 |
| Bioadhesive polymer | Methylcellulose (Methocel® A 15) | 10 | - |
| | Hypromellose (Methocel® E5) | - | 10 |
| Binder | Dextran 70 | 10 | 10 |
| Filler | Mannitol | 113 | 113 |
| Sweetener | Sucralose | 2 | 2 |
| Purified water | | 230 | 230 |
| Total weight of the solid dosage form | | 200 | 200 |
| Disintegration time of the solid dosage form (seconds) | | 45 | 26 |
| Density of the solid dosage form (mg/ml) | | 433 | 444 |
| Porosity of the solid dosage form (%) | | 56.7 | 55.6 |
| Hardness of the solid dosage form (N) | | Not Determined | Not Determined |
| Bioadhesion of the solid dosage form (min) | | Not Determined | Not Determined |

A mixture having the above formulations illustrated in Examples 11 and 12 was used to prepare a lyophilized bioadhesive dosage form according to the process details described in connection with Examples 9-10 above and tested according to the process details described in connection with Examples 1-3 above.

The resulting lyophilized bioadhesive patch had an excellent mechanical strength, bioadhesive characteristics, and disintegration time.

EXAMPLES 13-14

Examples 13-14 are compositions intended to relieve migraine or cluster headache attacks.

| Examples | | 13 | 14 |
|---|---|---|---|
| Components | | (mg) | (mg) |
| API | Sumatriptan succinate | 10 | 20 |
| Gelling agent | Poloxamer 407 | 15 | 15 |
| Bioadhesive polymer | Hypromellose (Methocel® E5) | 10 | - |
| | Methylcellulose (Methocel® A 15) | - | 15 |
| Binder | Dextran 70 | 10 | 10 |
| Filler | Isomaltidex | 10 | 10 |
| | Mannitol | - | 98 |
| | β Cyclodextrin | 95 | - |
| Sweetener | Sucralose | | |
| Buffered Solution* | | 200 | 220 |
| Total weight of the solid dosage form | | 150 | 170 |
| *Buffer solution is comprised of monobasic potassium phosphate, anhydrous dibasic sodium phosphate, sulfuric acid, sodium hydroxide, and purified water, wherein the pH of the solution is between about 5.5 and about 6.5. | | | |

A mixture having the above formulations illustrated in Examples 13 and 14 is used to prepare a lyophilized bioadhesive dosage form according to the process details described in connection with Examples 7-8 above.

EXAMPLES 15-16

Examples 15-16 are compositions intended for diurea, such as diabetes insipidus, nocturnal enuresis and urine incontinence.

| Examples | | 15 | 16 |
|---|---|---|---|
| Components | | (mg) | (mg) |
| API | Desmopressin | .06 | .120 |
| Gelling agent | Poloxamer 407 | 10 | 10 |
| Bioadhesive polymer | Methylcellulose (Methocel® A 15) | 7 | 7 |
| Binder | Dextran 70 | 5 | 5 |
| Filler | Mannitol | 57 | 57 |
| | Isomaltidex | 20 | 20 |
| Permeation Enhancer | Sodium Taurochoalte | .10 | .10 |
| Sweetener | Sucralose | .5 | .5 |
| Flavor | | .5 | .5 |
| Purified water | | 200 | 200 |
| Total weight of the solid dosage form | | 100 | 100 |
| Disintegration time of the solid dosage form (seconds) | | Not Determined | Not Determined |
| Density of the solid dosage form (mg/ml) | | 450 | 450 |

(continued)

| Examples | 15 | 16 |
|---|---|---|
| Components | (mg) | (mg) |
| Porosity of the solid dosage form (%) | 55 | 55 |
| Hardness of the solid dosage form (N) | Not Determined | Not Determined |
| Bioadhesion of the solid dosage form (min) | Not Determined | Not Determined |

A mixture having the above formulations illustrated in Examples 15 and 16 were used to prepare a lyophilized bioadhesive dosage form according to the process details described in connection with Examples 11-12 and tested according to the process details described in connection with Examples according to the process details described in connection with Examples 1-3 above.

The resulting lyophilized bioadhesive patch had an excellent mechanical strength, bioadhesive characteristics, and disintegration time.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

**Claims**

**1.** A lyophilized solid dosage form comprising:

(a) an active pharmaceutical ingredient (API),
(b) a gelling agent, and
(c) a bioadhesive polymer;

wherein said lyophilized solid dosage form adheres to an oral mucosal tissue for about 10 minutes up to about 180 minutes to release said API through said oral mucosal tissue.

**2.** The lyophilized solid dosage form according to claim 1, wherein said lyophilized solid dosage form adheres to an oral mucosal tissue for about 15 minutes up to about 180 minutes to release said API through said oral mucosal tissue.

**3.** The lyophilized solid dosage form according to claim 1 or 2, wherein said API is fentanyl, modafinil, ondansetron, granisetron, sumatriptan, tramadol, desmopressin, calcitonin, insulin, GLP-1, PPY, oxycontin, or taxol, or a mixture thereof.

**4.** The lyophilized solid dosage form according to claim 1, 2 or 3, wherein said API is present in an amount of from about 0.001% to about 70% relative to the total weight of the dosage form.

**5.** The lyophilized solid dosage form according to anyone of the preceding claims, wherein said gelling agent is a polyssaccaride, guar gum, arabic gum, tragacanth gum, xantham gum, carraghenan, pectin, starch, poloxamer 188, poloxamer 407, poloxamine, acrylic polymer, carbopol, or a mixture thereof.

**6.** The lyophilized solid dosage form according to anyone of the preceding claims, wherein said gelling agent is present in an amount from of about 0.5% to about 50% relative to the total weight of the dosage form.

**7.** The lyophilized solid dosage form according to anyone of the preceding claims, wherein said bioadhesive polymer is a cellulose derivative, cellulose, carboxymethyl cellulose, sodium carboxymethyl cellulose, methyl cellulose, hydroxypropyl cellulose, hydroxyl propyl methyl cellulose, hydroxyl ethyl cellulose, or a mixture thereof.

**8.** The lyophilized solid dosage form according to anyone of the preceding claims, wherein said bioadhesive polymer is present in an amount of from about 0.5% to about 30% relative to the total weight of the dosage form.

**9.** The lyophilized solid dosage form according to anyone of the preceding claims, further comprising an oil and an emulsifying agent, wherein said API is water insoluble and/or lipophilic.

**10.** The lyophilized solid dosage form according to claim 9, wherein said oil is Miglyol® 810-812-818, mono-triglyceride ester, di-triglycerides ester, fatty acid of glycerol, mineral oil, vegetable oil, a reformed vegetable oil, or a mixture thereof.

**11.** The lyophilized solid dosage form according to claim 9 or 10, wherein said emulsifying agent is polysorbate 20, polysorbate 60, polysorbate 80, sucroester 7, sucroester 11, sucroester 15, sorbitan, poloxamer, dioctyl sulfosuccinate, polyethoxyethers of fatty glycerides, lecithins, or a mixture thereof.

**12.** The lyophilized solid dosage form according to anyone of the preceding claims, further comprising at least one excipient.

**13.** The lyophilized solid dosage form according to claim 12, wherein said excipient is a binder, filler, permeation enhancer, buffer, defoaming agent, sweetener, coloring agent, flavoring agent, or a mixture thereof.

**14.** The lyophilized solid dosage form according to claim 13, wherein said binder is Dextran 70, povidone 12, povidone 17 or povidone 30, copovidone, gelatin, starch, pregelatinized starch, or a mixture thereof.

**15.** The lyophilized solid dosage form according to claim 13, wherein said filler is mannitol, dextrose, sorbitol, isomalt, glycocolle, cyclodextrin derivative, cyclodextrin, glucose, maltodextrine lactose, sucrose, calcium carbonate, or a mixture thereof.

**16.** The lyophilized solid dosage form according to claim 13, wherein said permeation enhancer is sodium salicylate, citric acid, sodium carbonate, sodium bicarbonate, sodium glyococholate, sodium taurocholate, or a mixture thereof.

**17.** The lyophilized solid dosage form according to claim 13, wherein said buffer is monobasic potassium phosphate, anhydrous dibasic sodium phosphate, sulfuric acid, sodium hydroxide, citric acid, sodium carbonate, sodium bicarbonate or a mixture thereof.

**18.** The lyophilized solid dosage form according to anyone of the preceding claims, with the proviso that when said gelling agent is poloxamer 407, said bioadhesive polymer is not chitosan or vice versa.

**19.** A lyophilized solid dosage form comprising:

(a) an active pharmaceutical ingredient (API),
(b) a gelling agent, and
(c) a bioadhesive polymer;

wherein said lyophilized solid dosage form exhibits at least one of the following characteristics: the density of said lyophilized solid dosage form is from about 100 mg/ml to about 900 mg/ml, the porosity of said lyophilized solid dosage form is from about 10% to about 90%, and the hardness of said lyophilized solid dosage form is from about 4.5 N to about 100 N.

**20.** The lyophilized solid dosage form according to claim 19, wherein said lyophilized solid dosage form exhibits at least two of the following characteristics: a density is from about 100 mg/ml to about 900 mg/ml, a porosity is from about 10% to about 90%, and a hardness is from about 4.5 to about 100 N.

**21.** The lyophilized solid dosage form according to claim 19 or 20, wherein said density ranges from about 300 mg/ml to about 600 mg/ml.

**22.** The lyophilized solid dosage form according to claim 19, 20 or 21, wherein said porosity ranges from about 30% to about 60%.23. The lyophilized solid dosage form according to anyone of claims 19 to 22, wherein said hardness ranges from about 10N to about 45 N.

**23.** The lyophilized solid dosage form according to anyone of claims 19 to 22, wherein said dosage form has a maximum diameter of from about 10 to about 16 mm and a thickness of from about 1 to about 3 mm.

**24.** A lyophilized solid dosage form according to anyone of the preceding claims, wherein said API comprises:

(i) a non-coated API,and
(ii) a coated API.

**25.** A method of preparing a lyophilized solid dosage form for transmucosal delivery of at least one API comprising the steps of:

(a) preparing a suspension containing a gelling agent, a bioadhesive polymer, and an API,
(b) depositing said suspension from step (a) into a mould,
(c) freezing and then lyophilizing said suspension of step (b) in said mould to form a lyophilized solid dosage form, and
(d) sealing said lyophilized solid dosage form in said mould.

**26.** The method according to claim 25, wherein said suspension containing said gelling agent, said bioadhesive polymer and said API is prepared by:

(a) dissolving said gelling agent in water at a temperature of about 10°C to about 20°C,
(b) dissolving said bioadhesive polymer in water at a temperature of about 50°C to about 70°C,
(c) mixing said suspensions from steps (a) and (b), and
(d) adding API to said suspension from step (c) and mixing said suspension until the suspension is substantially homogeneous.

**27.** The method according to claims 25 or 26, wherein said step (c) of mixing is performed at a temperature between about 10°C and about 30°C.

**28.** The method according to claims 25, 26 or 27, wherein said step (c) of mixing is performed at a temperature between about 15°C and about 25°C.

**29.** A method of preparing a lyophilized solid dosage form for transmucosal delivery of at least one API comprising the steps of:

(a) preparing an aqueous suspension containing at least one hydrophilic compound,
(b) preparing an oil suspension containing at least one API and at least one lipophilic compound,
(c) mixing said suspension phase of step (a) and said oil suspension from step (b) to form an emulsion,
(d) depositing said emulsion from step (c) into a mould,
(e) freezing and then lyophilizing said emulsion of step (d) in said mould to form a lyophilized solid dosage form, and
(f) sealing said lyophilized solid dosage form in said mould.

**30.** The method according to claim 29, wherein said hydrophilic compound is a gelling agent, bioadhesive polymer, binder, and filler.

**31.** The method according to claim 29 or 30, wherein said lipophilic compound is an emulsifying agent, sweetener, and flavoring agent.

**32.** Use of a lyophilized solid dosage form according to anyone of claims 1 to 24, containing a therapeutically effective amount of at least one API for the preparation of a medicament for treating a patient, said medicament being suitable for:

(a) placing said lyophilized solid dosage form onto an oral mucosal tissue of a patient in need of treatment; and
(b) allowing said lyophilized solid dosage form to release said therapeutically effective amount of said at least one API through said oral mucosal tissue while adhering to said oral mucosal tissue.

European Patent Office

# EUROPEAN SEARCH REPORT

Application Number
EP 07 29 0443

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/084530 A1 (RAO PAVULURI V [IN] ET AL) 21 April 2005 (2005-04-21)<br>* page 1, paragraph 1 *<br>* page 3, paragraph 30 *<br>* example 2 *<br>----- | 1-8, 12-17, 19-23, 25-27, 29,32 | INV.<br>A61K9/00<br>A61K9/19<br>A61K9/14<br>A61K31/4178<br>A61K31/135<br>A61K31/4045<br>A61K38/11 |
| X | WO 2007/034287 A (UNIV THE WITWATERSRAND JOHANNE [ZA]; PATEL RUPAL [ZA]; PILLAY VINESS [) 29 March 2007 (2007-03-29)<br>* page 2, line 27 - page 3, line 12 *<br>* page 4, line 16 - line 22 *<br>* page 15, line 4 - line 10 *<br>----- | 1,2, 4-15, 19-28,32 | |
| X | US 2003/143277 A1 (AMEYE DIETER [BE] ET AL) 31 July 2003 (2003-07-31)<br>* example 6 *<br>----- | 1-6,8, 12-15, 19-23, 25-28,32 | TECHNICAL FIELDS SEARCHED (IPC) |
| X | WO 2004/041118 A (UMD INC [US]) 21 May 2004 (2004-05-21)<br>* page 52, line 15 - line 25 *<br>* page 54, line 25 - page 55 *<br>* table 3 *<br>* page 14, line 29 *<br>* page 15, line 9 - page 17, line 8 *<br>----- | 1,2,4-8, 12-15, 19-23, 25,26,32 | A61K |

-/--

The present search report has been drawn up for all claims

| Place of search | Date of completion of the search | Examiner |
|---|---|---|
| Munich | 20 November 2007 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

European Patent Office

# EUROPEAN SEARCH REPORT

Application Number
EP 07 29 0443

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/077336 A (KOREA INST SCIENCE TECHNOLOGY [KR]; CHUNG HESSON [KR]; JEONG SEO-YOUNG) 25 August 2005 (2005-08-25)<br>* page 1, line 11 - page 2, line 20 *<br>* examples 32,53 *<br>* page 11, line 12 - line 24 *<br>----- | 1-6, 8-15, 18-23, 25-32 | |
| A | WO 2006/103657 A (DEXCEL PHARMA TECHNOLOGIES LTD [IL]; PINHASI ADEL [IL]; GOMBERG MILA [])<br>5 October 2006 (2006-10-05)<br>* example 26 *<br>* page 1, paragraph 1 *<br>----- | 1-32 | |

TECHNICAL FIELDS SEARCHED (IPC)

The present search report has been drawn up for all claims

| Place of search | Date of completion of the search | Examiner |
|---|---|---|
| Munich | 20 November 2007 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

## ANNEX TO THE EUROPEAN SEARCH REPORT ON EUROPEAN PATENT APPLICATION NO. EP 07 29 0443

This annex lists the patent family members relating to the patent documents cited in the above-mentioned European search report. The members are as contained in the European Patent Office EDP file on
The European Patent Office is in no way liable for these particulars which are merely given for the purpose of information.

20-11-2007

| Patent document cited in search report | Publication date | Patent family member(s) | Publication date |
|---|---|---|---|
| US 2005084530 A1 | 21-04-2005 | NONE | |
| WO 2007034287 A | 29-03-2007 | NONE | |
| US 2003143277 A1 | 31-07-2003 | CN 1646105 A | 27-07-2005 |
| | | EP 1469837 A1 | 27-10-2004 |
| | | JP 2005526021 T | 02-09-2005 |
| | | WO 03063839 A1 | 07-08-2003 |
| WO 2004041118 A | 21-05-2004 | AU 2003286796 A1 | 07-06-2004 |
| | | BR 0315194 A | 23-08-2005 |
| | | CA 2504283 A1 | 21-05-2004 |
| | | EP 1556001 A2 | 27-07-2005 |
| | | JP 2006512409 T | 13-04-2006 |
| | | MX PA05004578 A | 26-07-2005 |
| | | NZ 540318 A | 28-09-2007 |
| | | US 2004151774 A1 | 05-08-2004 |
| WO 2005077336 A | 25-08-2005 | KR 20050081092 A | 18-08-2005 |
| WO 2006103657 A | 05-10-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6200604 B
- US 6974590 B, Shin
- US 4616047 A, Lafon
- US 5843347 A, Nguyen
- US 3855712 A, Blonde
- US 4490407 A
- US 3939260 A
- US 6024981 A
- US 4949588 A
- US 5178878 A
- US 5055306 A


### Non-patent literature cited in the description


- Mucoadhesive and Physico-chemical Characterization of Carbopol-Poloxamer Gels Containing Triamcinolone Acetonide. *Drug Development and Industrial Pharmacy,* 2000, vol. 26 (3), 307-312
- **CAFFAGGI et al.** Preparation and Evaluation of a Chitosan Salt-Poloxamer 407 Based Matrix for Buccal Drug Delivery. *Journal of Controlled Release,* 2005, vol. 102, 159-169
- **LIEBERMAN.** Pharmaceutical Dosage Form: Tablets. 1989, vol. 1, 732-36